# EUROPÄISCHE PATENTSCHRIFT

(11) **EP 3 993 769 B1**
(45) Veröffentlichungstag und Bekanntmachung des Hinweises auf die Patenterteilung: **30.08.2023**
(21) Anmeldenummer: 20736330.0
(22) Anmeldetag: 02.07.2020
(51) Int. Cl.: A61K 9/00, A61K 31/135

(54) **ORALER DÜNNFILM**
ORAL THIN FILM
FILM MINCE D'HYGIÈNE BUCCO-DENTAIRE

(30) Priorität: 02.07.2019 DE 102019117870
(43) Veröffentlichungstag der Anmeldung: 11.05.2022
(73) Patentinhaber: LTS LOHMANN Therapie-Systeme AG, 56626 Andernach (DE)
(72) Erfinder: MÜLLER, Markus, 53840 Troisdorf (DE); LINN, Michael, 55596 Waldböckelheim (DE)
(74) Vertreter: Meissner Bolte Partnerschaft mbB
(86) Internationale Anmeldenummer: PCT/EP2020/068598
(87) Internationale Veröffentlichungsnummer: WO 2021/001461

(56) Entgegenhaltungen:
- WO-A1-2014/020155
- WO-A2-2011/057714
- DE-A1-102017 129 012
- US-A- 3 972 995

## Beschreibung

Die vorliegende Erfindung betrifft einen oralen Dünnfilm, ein Verfahren zu dessen Herstellung und dessen Verwendung als Arzneimittel.

Orale Dünnfilme (OTF) sind dünne, mindestens einen pharmazeutisch aktiven Wirkstoff enthaltende Filme, die direkt in den Mundraum gelegt oder an die Mundschleimhaut angelegt werden und sich dort auflösen. Dabei handelt es sich insbesondere um dünne wirkstoffhaltige Filme auf Polymerbasis, die, wenn sie auf eine Schleimhaut, insbesondere die Mundschleimhaut, aufgebracht werden, den Wirkstoff direkt in diese abgeben. Die sehr gute Durchblutung der Mundschleimhaut sorgt für einen schnellen Übergang des Wirkstoffs in den Blutkreislauf. Dieses Darreichungssystem hat den Vorteil, dass der Wirkstoff größtenteils durch die Schleimhaut resorbiert wird und damit der "First-Pass Metabolismus", der bei der konventionellen Darreichungsform eines Wirkstoffs in Tablettenform auftritt, vermieden wird. Der Wirkstoff kann in dem Film gelöst, emulgiert oder dispergiert werden.

Aus dem Stand der Technik bekannte orale Dünnfilme haben den Nachteil, dass die Menge des darin enthaltenen mindestens einen pharmazeutisch aktiven Wirkstoffs im Wesentlichen durch die Sättigungskonzentration des mindestens einen pharmazeutisch aktiven Wirkstoffs in dem oralen Dünnfilm begrenzt ist. Es gab jedoch auch Versuche orale Dünnfilme bereitzustellen, die den mindestens einen pharmazeutisch aktiven Wirkstoff in einer Konzentration die größer ist als die Sättigungskonzentration des mindestens einen pharmazeutisch aktiven Wirkstoffs in dem oralen Dünnfilm enthalten. In derartigen oralen Dünnfilmen liegt der Wirkstoff aber im Wesentlichen ungelöst vor, weswegen diese oralen Dünnfilme auch als Suspensions-OTF bekannt sind. Diese Suspensions-OTFs haben jedoch den Nachteil, dass der Wirkstoff bei der Masseherstellung, der Beschichtung und/oder der Trocknung des oralen Dünnfilms unkontrolliert und inhomogen auskristallisieren kann und somit zu inhomogenen Wirkstofflaminaten führen kann, insofern der Wirkstoff in der Beschichtungsmasse (teilweise) gelöst vorliegt. Diese inhomogenen Wirkstofflaminate sind in mehrerlei Hinsicht nachteilig. So kann deren mechanische Integrität durch die inhomogene Kristallisation des Wirkstoffs herabgesetzt sein und die Bioverfügbarkeit des Wirkstoffs kann durch die inhomogene Kristallisation des Wirkstoffs beeinträchtigt sein.

So offenbart die WO 2014020155 A1 ein OTF auf Basis einer festen Suspension.

DE 10 2017 129012 A1 offenbart einen oralen Dünnfilm umfassend mindestens ein Polymer (Cellulosederivat) und mindestens einen pharmazeutisch aktiven Wirkstoff. Es wird kein Material offenbart, das als Kristallisationskeim für den Wirkstoff dient.

Die Aufgabe der vorliegenden Erfindung besteht darin, vorstehend genannte Nachteile des Standes der Technik zu beheben. Insbesondere besteht die Aufgabe der vorliegenden Erfindung darin, einen oralen Dünnfilm mit einem relativ hohen Wirkstoffgehalt, d.h. einer Wirkstoffkonzentration, die größer ist als die Sättigungskonzentration des mindestens einen pharmazeutisch aktiven Wirkstoffs in dem oralen Dünnfilm, bereitzustellen, bei dem der mindestens eine pharmazeutisch aktiven Wirkstoff nicht inhomogen auskristallisiert, sondern als eine im Wesentlichen homogene Kristallphase in dem oralen Dünnfilm vorliegt. Zudem besteht eine weitere Aufgabe der vorliegenden Erfindung darin, ein wirtschaftlich akzeptables Verfahren zur Herstellung eines solchen oralen Dünnfilms bereitzustellen.

Obige Aufgabe wird durch einen oralen Dünnfilm nach Anspruch 1 gelöst, der mindestens ein Polymer und mindestens einen pharmazeutisch aktiven Wirkstoff umfasst, wobei die Konzentration des mindestens einen pharmazeutisch aktiven Wirkstoffs in dem oralen Dünnfilm größer ist als die Sättigungskonzentration des mindestens einen pharmazeutisch aktiven Wirkstoffs in dem oralen Dünnfilm und wobei der orale Dünnfilm mindestens ein Material, das als Kristallisationskeim für den mindestens einen pharmazeutisch aktiven Wirkstoff dient, umfasst.

Ein solcher oraler Dünnfilm hat den Vorteil, dass während der Trocknung des oralen Dünnfilms an dem Punkt, an dem die Sättigungskonzentration des mindestens einen pharmazeutisch aktiven Wirkstoffs in dem oralen Dünnfilm überschritten wird, dieser mindestens eine pharmazeutisch aktiven Wirkstoffs nicht unkontrolliert und inhomogen auskristallisiert, da das mindestens ein Material, das als Kristallisationskeim für den mindestens einen pharmazeutisch aktiven Wirkstoff dient, eine homogene und kontrollierte Kristallisation des mindestens einen pharmazeutisch aktiven Wirkstoffs in dem oralen Dünnfilm bewirkt.

Kristallisationskeime oder -kerne sind feindisperse oder makroskopische, feste Partikel in einer fluiden Phase, die die Kristallisation, also die Bildung von Kristallen, erleichtern.

Die Konzentration des mindestens einen pharmazeutisch aktiven Wirkstoffs in dem oralen Dünnfilm ist größer als die Sättigungskonzentration des mindestens einen pharmazeutisch aktiven Wirkstoffs in dem oralen Dünnfilm. Dabei wird die Sättigung im Aggregatszustand fest-in-fest verstanden. D.h. die Konzentration des festen Wirkstoffs, in dem getrockneten, festen oralen Dünnfilm.

Unter Sättigungskonzentration wird die Konzentration des mindestens einen pharmazeutisch aktiven Wirkstoffs in dem oralen Dünnfilm angesehen, bei der der Wirkstoff nach Ausstreichen der Mischung eigenständig rekristallisiert. Die Rekristallisation kann dabei spontan nach dem Ausstreichen erfolgen. Es ist dem Fachmann bekannt, dass eine solche Rekristallisation aber auch zeitverzögert nach dem Ausstreichen erfolgen kann.

Der erfindungsgemäße orale Dünnfilm ist vorzugsweise dadurch gekennzeichnet, dass das Material, das als Kristallisationskeim für dem mindestens einen pharmazeutisch aktiven Wirkstoff dient in einer Menge von 0,01 bis 20 Gew.-%, bevorzugt 0,01 bis 10 Gew.-%, besonders bevorzugt 0,01 bis 5 Gew.-%, bezogen auf das Gesamtgewicht des oralen Dünnfilms, in dem oralen Dünnfilm enthalten ist.

Liegt das Material, das als Kristallisationskeim für den mindestens einen pharmazeutisch aktiven Wirkstoff dient, in geringeren Mengen vor, so sind nicht genügend Kristallisationskeime vorhanden, um eine homogene Kristallisation sicherzustellen.

Liegt das Material, das als Kristallisationskeim für den mindestens einen pharmazeutisch aktiven Wirkstoff dient, in größeren Mengen vor, so können die Filmeigenschaften nachteilig beeinflusst werden.

Der erfindungsgemäße orale Dünnfilm ist bevorzugt dadurch gekennzeichnet, dass das Material, das als Kristallisationskeim für dem mindestens einen pharmazeutisch aktiven Wirkstoff dient kleine im Milieu der Herstellungsmasse unlösliche Partikel umfasst.

Unter klein wird in diesem Zusammenhang eine mittlere Partikelgröße von 5 bis 100 µm, vorzugsweise von 20 bis 50 µm verstanden, wobei die mittlere Partikelgröße den zum Anmeldetag gültigen Produktdatenblättern der jeweiligen Hersteller entnommen wird.

Der erfindungsgemäße orale Dünnfilm ist ferner besonders bevorzugt dadurch gekennzeichnet, dass das Material, das als Kristallisationskeim für den mindestens einen pharmazeutisch aktiven Wirkstoff dient, amorphes pyrogenes Siliciumdioxid umfasst.

Amorphes pyrogenes Siliciumdioxid (SiOz), auch als amorphe pyrogene Kieselsäure (oder englisch als fumed silica) bezeichnet, ist ein synthetisch hergestelltes, kolloides Siliciumdioxid. Es besteht im Wesentlichen vollständig aus amorphen Siliciumdioxid-Partikeln, die zu größeren Einheiten aggregiert sind.

Geeignete amorphe pyrogene Siliciumdioxide sind beispielsweise aus der Serie mit dem Handelsnamen "Aerosil^{®}" von Evonik bekannt.

Außerdem geeignet als Materialen, die als Kristallisationskeime für den mindestens einen pharmazeutisch aktiven Wirkstoff dienen, sind TiO₂, Eisenoxid(e), Aluminiumtrioxid und/oder Magnesium-Stearat.

In einer bevorzugten Ausführungsform ist der erfindungsgemäße orale Dünnfilm dadurch gekennzeichnet, dass das mindestens eine Polymer ein wasserlösliches und/oder wasserquellbares Polymer umfasst.

Wasserlösliche/wasserquellbare Polymere umfassen chemisch sehr unterschiedliche, natürliche oder synthetische Polymere, deren gemeinsames Merkmal ihre Löslichkeit/Quellbarkeit in Wasser oder wässrigen Medien ist.

Voraussetzung ist, dass diese Polymere eine für die Wasserlöslichkeit/Wasserquellbarkeit ausreichende Anzahl an hydrophilen Gruppen besitzen und nicht vernetzt sind. Die hydrophilen Gruppen können nichtionisch, anionisch, kationisch und/oder zwitterionisch sein.

Vorzugsweise ist das mindestens eine Polymer in dem erfindungsgemäßen oralen Dünnfilm ausgewählt aus der Gruppe bestehend aus Stärke, Dextranen, Carboxymethylcellulose, Hydroxypropylcellulose, Hydroxyethylcellulose, Hydroxypropylmethylcellulose, Hydroxypropylethylcellulose, Natriumcarboxymethylcellulose, Ethyl- oder Propylcellulose, Polyacrylsäuren, Polyacrylaten, Polyvinylpyrrolidonen, Polyvinylalkoholen, Polyethylenoxidpoylmeren, Polyacrylamiden, Polyethylenglycolen, Gelatine, Collagen, Alginaten, Pektin, Pullulan, Traganth, Chitosan, Alginsäure, Arabinogalaktan, Galaktomannan, Agar, Agarose, Carrageen, natürlichen Gummen, sowie Mischungen davon.

In einer bevorzugten Ausführungsform ist der erfindungsgemäße orale Dünnfilm dadurch gekennzeichnet, dass das mindestens eine Polymer in einer Menge von 30 bis 80 Gew.-%, bezogen auf das Gesamtgewicht des oralen Dünnfilms, in dem oralen Dünnfilm enthalten ist.

Der in dem erfindungsgemäßen oralen Dünnfilm enthaltene mindestens eine pharmazeutisch aktive Wirkstoff ist nicht beschränkt. Beispiele für geeignet Wirkstoffklassen umfassen Hypnotica, Sedativa, Antiepileptica, Weckaminen, Psychoneurotropica, Neuro-Muskelblockern, Antispasmodica, Antihistaminica, Antiallergica, Cardiotonica, Antiarrhythmica, Diuretica, Hypotensiva, Vasopressoren, Antidepressiva, Antitussiva, Expectorantia, Thyroidhormonen, Sexualhormonen, Antidiabetica, Antitumor-Wirkstoffen, Antibiotica, Chemotherapeutica und Narcotica.

Besonders bevorzugt umfasst der mindestens eine pharmazeutisch aktive Wirkstoff in dem erfindungsgemäßen oralen Dünnfilm Ketamin und/oder ein pharmazeutisch akzeptables Salz, bevorzugt Ketamin·HCl, davon.

Unter Ketamin wird (S)-(±)-2-(2-Chlorphenyl)-2-(methylamino)cyclohexan-1-on ((S)-Ketamin), (R)-(±)-2-(2-Chlorphenyl)-2-(methylamino)cyclohexan-1-on ((R)-Ketamin), sowie das Racemat (RS)-(±)-2-(2-Chlorphenyl)-2-(methylamino)cyclohexan-1-on verstanden.

Ganz besonders bevorzugt umfasst der der mindestens eine pharmazeutisch aktive Wirkstoff in dem erfindungsgemäßen oralen Dünnfilm (S)-Ketamin und/oder ein pharmazeutisch akzeptables Salz, bevorzugt (S)-Ketamin·HCl, davon.

Bevorzugt umfasst der mindestens eine pharmazeutisch aktive Wirkstoff in dem erfindungsgemäßen oralen Dünnfilm Adrenalin ((R)-4-[1-Hydroxy-2-(methylamino)ethyl]benzen-1,2-diol), vorzugsweise als Hydrogentartrat.

Der erfindungsgemäße orale Dünnfilm ist vorzugsweise dadurch gekennzeichnet, dass der orale Dünnfilm mindestens einen Hilfsstoff ausgewählt aus der Gruppe, umfassend Farbstoffe, Aromastoffe, Süßstoffe, geschmacksmaskierende Mittel, Emulgatoren, Enhancer, pH-Regulatoren, Feuchthaltemittel, Konservierungsmittel und/oder Antioxidationsmittel, umfasst.

Jeder dieser Hilfsstoffe ist vorzugsweise jeweils in einer Menge von etwa 0,1 bis 10 Gew.-%, bezogen auf das Gesamtgewicht des oralen Dünnfilms, in dem oralen Dünnfilm enthalten.

Der erfindungsgemäße orale Dünnfilm ist vorzugsweise dadurch gekennzeichnet, dass das Flächengewicht des oralen Dünnfilms etwa 50 bis 300 g/m², vorzugsweise etwa 100 bis 200 g/m², beträgt.

Besonders bevorzugt umfasst der erfindungsgemäße orale Dünnfilm (S)-Ketamin, bevorzugt in einer Menge von 40 bis 50 Gew.-%, bezogen auf das Gesamtgewicht des oralen Dünnfilms, mindestens eine Hydroxypropylmethylcellulose, bevorzugt in einer Menge von 10 bis 50 Gew.-%, bezogen auf das Gesamtgewicht des oralen Dünnfilms, und ein pyrogenes amorphes Siliciumdioxid, bevorzugt in einer Menge von 0,1 bis 2 Gew.-%, bezogen auf das Gesamtgewicht des oralen Dünnfilms.

Die vorliegende Erfindung betrifft ferner ein Verfahren zur Herstellung des vorstehend beschriebenen oralen Dünnfilms.

Das Verfahren umfasst die Schritte
a) Herstellen einer Suspension bzw. Lösung, umfassend das mindestens eine Polymer, den mindestens einen pharmazeutisch aktiven Wirkstoff und das Material, das als Kristallisationskeim für den mindestens einen pharmazeutisch aktiven Wirkstoff dient, und
b) Ausstreichen und Trocknen der in Schritt a) erhaltenen Suspension bzw. Lösung, um einen Dünnfilm, vorzugsweise mit einem Flächengewicht von etwa 50 bis 300 g/m², zu erhalten.

Die Menge des in der Suspension bzw. Lösung in Schritt a) vorhandenen mindestens einen pharmazeutisch aktiven Wirkstoffs ist vorzugsweise so bemessen, dass der Wirkstoff in dem nach der Trocknung in Schritt b) erhaltenen oralen Dünnfilm in einer Konzentration vorliegt, die größer ist als die Sättigungskonzentration des mindestens einen pharmazeutisch aktiven Wirkstoffs in dem nach der Trocknung in Schritt b) erhaltenen oralen Dünnfilm.

Das in Verfahrensschritt a) eingesetzte Lösungsmittel ist vorzugsweise ein wässriges Lösungsmittel.

Vorzugsweise kann die Suspension bzw. Lösung in Verfahrensschritt a) erwärmt werden.

Es ist folglich besonders bevorzugt, wenn der mindestens eine pharmazeutisch aktive Wirkstoff in Schritt a) im Wesentlichen vollständig gelöst vorliegt.

Unter "im Wesentlichen vollständig" wird hierbei verstanden, dass mindestens 85%, bevorzugt mindestens 90%, ganz besonders bevorzugt mindestens 95% und am meisten bevorzugt mindestens 99% des mindestens einen pharmazeutisch aktiven Wirkstoffs in gelöster Form vorliegt.

Es ist ferner bevorzugt, dass der mindestens eine pharmazeutisch aktive Wirkstoff bei der Trocknung in Schritt b) im Wesentlichen bis unter die Sättigungsgrenze (Sättigungskonzentration fest-in fest) auskristallisiert. Besonders bevorzugt liegt der mindestens eine pharmazeutisch aktive Wirkstoff im Wesentlichen vollständig und homogen verteilt in kristalliner Form in dem oralen Dünnfilm vor.

Unter "im Wesentlichen" wird hierbei verstanden, dass mindestens 85%, bevorzugt mindestens 90%, ganz besonders bevorzugt mindestens 95% und am meisten bevorzugt mindestens 99% des mindestens einen pharmazeutisch aktiven Wirkstoffs in kristalliner Form, vorzugsweise homogen verteilt, in dem oralen Dünnfilm vorliegt.

Die vorliegende Erfindung betrifft weiterhin einen oralen Dünnfilm erhältlich nach dem vorstehend beschriebenen Verfahren.

Außerdem betrifft die vorliegende Erfindung einen oralen Dünnfilm, wie vorstehend beschrieben oder erhältlich nach dem vorstehend beschriebenen Verfahren, als Arzneimittel.

Die vorliegende Erfindung betrifft zudem einen oralen Dünnfilm, wie vorstehend beschrieben oder erhältlich nach dem vorstehend beschriebenen Verfahren als Arzneimittel zur Verwendung in der Behandlung von Depressionen, insbesondere zu Reduzierung des Suizidrisikos und/oder zur Verwendung als Allgemeinanästhetikum, vorzugsweise zur Einleitung und Durchführung einer Vollnarkose oder als Ergänzung bei Regionalanästhesien und/oder als Analgetikum.

Die vorstehend für den erfindungsgemäßen Dünnfilm aufgeführten bevorzugten Ausführungsformen gelten auch für das erfindungsgemäße Verfahren, den mit diesem erhaltenen oralen Dünnfilm und für dessen Verwendung als Arzneimittel.

### Beschreibung der Figuren

Figur 1 zeigt einen erfindungsgemäßen Dünnfilm gemäß der in Tabelle 1 aufgeführten Rezeptur nach der Trocknung.
Figur 2 zeigt einen Dünnfilm gemäß der in Tabelle 1 aufgeführten Vergleichsbeispiel nach der Trocknung.

Die Erfindung wird nachfolgend anhand von nicht beschränkenden Beispielen näher erläutert.

### Beispiele

### Beispiel 1:

| Inhaltsstoff | Funktion | Anteil [Gew.-%] | |
|---|---|---|---|
| | | | Vergleichsbeispiel |
| (S)-Ketamin | Wirkstoff | 41,0 | 41,0 |
| HPMC 603 (3 mPas)¹ | Polymer | 39,0 | 39,5 |
| HPMC 60SH50 (50 mPas)¹ | Polymer | 10,0 | 10,0 |
| Glycerin | Feuchthaltemittel | 3,5 | 3,5 |
| Succralose | Geschmackskorrigenz | 1,0 | 1,0 |
| Saccharin Na | Geschmackskorrigenz | 2,0 | 2,0 |
| Cerry Flavour EU | Geschmackskorrigenz | 3,0 | 3,0 |
| Aerosil 200² | Kristallisationskeim | 0,5 | -- |
| Wasser | Restfeuchte | 8 | 8 |

| | | | |
|---|---|---|---|
| ¹: Hydroxypropylmethylcellulosen mit unterschiedlichen Viskositäten, wobei diese eine Viskosität von etwa 3 bzw. etwa 50 mPas (gemessen nach USP Monographie <911> Methode 1, aus 2012) aufweisen. ²: Pyrogenes amorphes SiO₂ von Evonik. | | | |

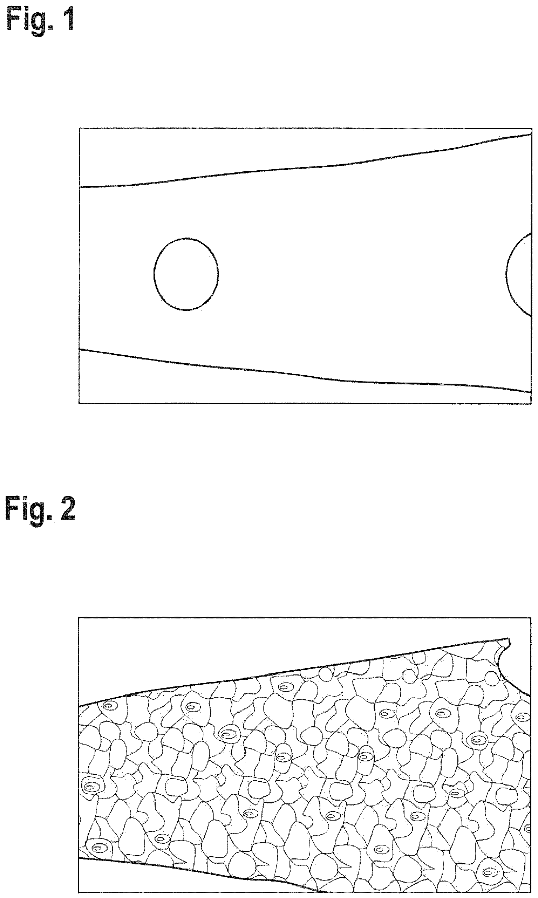

Der erfindungsgemäße orale Dünnfilm weist ein homogenes Erscheinungsbild auf. Der Wirkstoff liegt in kristalliner Form homogen verteilt in dem oralen Dünnfilm vor (siehe Figur 1).

Der orale Dünnfilm gemäß der Rezeptur des Vergleichsbeispiels weist ein äußerst inhomogenes Erscheinungsbild auf. Der Wirkstoff ist inhomogen auskristallisiert, sodass der orale Dünnfilm viele Bruchlinien aufweist (siehe Figur 2).

## Patentansprüche

1. Oraler Dünnfilm umfassend mindestens ein Polymer und mindestens einen pharmazeutisch aktiven Wirkstoff, wobei die Konzentration des mindestens einen pharmazeutisch aktiven Wirkstoffs in dem oralen Dünnfilm größer ist als die Sättigungskonzentration des mindestens einen pharmazeutisch aktiven Wirkstoffs in dem oralen Dünnfilm, **dadurch gekennzeichnet, dass** der orale Dünnfilm mindestens ein Material, das als Kristallisationskeim für den mindestens einen pharmazeutisch aktiven Wirkstoff dient, umfasst.

2. Oraler Dünnfilm gemäß Anspruch 1, **dadurch gekennzeichnet, dass** das Material, das als Kristallisationskeim für dem mindestens einen pharmazeutisch aktiven Wirkstoff dient, in einer Menge von 0,01 bis 20 Gew.-%, bezogen auf das Gesamtgewicht des oralen Dünnfilms, in dem oralen Dünnfilm enthalten ist.

3. Oraler Dünnfilm gemäß irgendeinem der vorhergehenden Ansprüche,
**dadurch gekennzeichnet, dass** das Material, das als Kristallisationskeim für dem mindestens einen pharmazeutisch aktiven Wirkstoff dient, amorphes pyrogenes Siliciumdioxid umfasst.

4. Oraler Dünnfilm gemäß irgendeinem der vorhergehenden Ansprüche,
**dadurch gekennzeichnet, dass** das mindestens eine Polymer ein wasserlösliches und/oder wasserquellbares Polymer umfasst.

5. Oraler Dünnfilm gemäß irgendeinem der vorhergehenden Ansprüche,
**dadurch gekennzeichnet, dass** das mindestens eine Polymer ausgewählt ist aus der Gruppe bestehend aus Stärke, Dextranen, Carboxymethylcellulose, Hydroxypropylcellulose, Hydroxyethylcellulose, Hydroxypropylmethylcellulose, Hydroxypropylethylcellulose, Natriumcarboxymethylcellulose, Ethyl- oder Propylcellulose, Polyacrylsäuren, Polyacrylaten, Polyvinylpyrrolidonen, Polyvinylalkoholen, Polyethylenoxidpoylmeren, Polyacrylamiden, Polyethylenglycolen, Gelatine, Collagen, Alginaten, Pektin, Pullulan, Traganth, Chitosan, Alginsäure, Arabinogalaktan, Galaktomannan, Agar, Agarose, Carrageen, natürlichen Gummen, sowie Mischungen davon.

6. Oraler Dünnfilm gemäß irgendeinem der vorhergehenden Ansprüche,
**dadurch gekennzeichnet, dass** das mindestens eine Polymer in einer Menge von 30 bis 80 Gew.-%, bezogen auf das Gesamtgewicht des oralen Dünnfilms, in dem oralen Dünnfilm enthalten ist.

7. Oraler Dünnfilm gemäß irgendeinem der vorhergehenden Ansprüche,
**dadurch gekennzeichnet, dass** der pharmazeutisch aktive Wirkstoff ausgewählt ist aus der Gruppe, bestehend aus Hypnotica, Sedativa, Antiepileptica, Weckaminen, Psychoneurotropica, Neuro-Muskelblockern, Antispasmodica, Antihistaminica, Antiallergica, Cardiotonica, Antiarrhythmica, Diuretica, Hypotensiva, Vasopressoren, Antidepressiva, Antitussiva, Expectorantia, Thyroidhormonen, Sexualhormonen, Antidiabetica, Antitumor-Wirkstoffen, Antibiotica, Chemotherapeutica und Narcotica.

8. Oraler Dünnfilm gemäß irgendeinem der vorhergehenden Ansprüche,
**dadurch gekennzeichnet, dass** der mindestens eine pharmazeutisch aktive Wirkstoff Ketamin, vorzugsweise (S)-Ketamin, oder ein pharmazeutisch akzeptables Salz davon umfasst.

9. Oraler Dünnfilm gemäß irgendeinem der vorhergehenden Ansprüche,
**dadurch gekennzeichnet, dass** der orale Dünnfilm ferner mindestens einen Hilfsstoff ausgewählt aus der Gruppe, umfassend Farbstoffe, Aromastoffe, Süßstoffe, geschmacksmaskierende Mittel, Emulgatoren, Enhancer, pH-Regulatoren, Feuchthaltemittel, Konservierungsmittel und/oder Antioxidationsmittel, umfasst.

10. Verfahren zur Herstellung eine oralen Dünnfilms gemäß irgendeinem der Ansprüche 1 bis 9, umfassend die Schritte:
a) Herstellen einer Suspension bzw. Lösung, umfassend das mindestens eine Polymer, den mindestens einen pharmazeutisch aktiven Wirkstoff und das Material, das als Kristallisationskeim für den mindestens einen pharmazeutisch aktiven Wirkstoff dient, und
b) Ausstreichen und Trocknen der in Schritt a) erhaltenen Suspension bzw. Lösung, um einen Dünnfilm, vorzugsweise mit einem Flächengewicht von etwa 50 bis 300 g/m², zu erhalten.

11. Verfahren gemäß Anspruch 10, wobei der mindestens eine pharmazeutisch aktive Wirkstoff in Schritt a) im Wesentlichen vollständig gelöst vorliegt.

12. Verfahren gemäß irgendeinem der Ansprüche 10 bis 11, wobei der mindestens eine pharmazeutisch aktive Wirkstoff bei der Trocknung in Schritt b) bis unter die Sättigungsgrenze auskristallisiert.

13. Oraler Dünnfilm erhältlich nach dem Verfahren gemäß irgendeinem der Ansprüche 10 bis 12.

14. Oraler Dünnfilm gemäß irgendeinem der Ansprüche 1 bis 9 und 13 zur Verwendung als Arzneimittel.

## Claims

1. An oral thin film comprising at least one polymer and at least one pharmaceutically active agent, wherein the concentration of the at least one pharmaceutically active agent in the oral thin film is greater than the saturation concentration of the at least one pharmaceutically active agent in the oral thin film, **characterised in that** the oral thin film comprises at least one material which serves as crystal nucleus for the at least one pharmaceutically active agent.

2. The oral thin film according to claim 1, **characterised in that** the material which serves as crystal nucleus for the at least one pharmaceutically active agent is contained in the oral thin film in an amount of 0.01 to 20 wt.% in relation to the total weight of the oral thin film.

3. The oral thin film according to any one of the preceding claims, **characterised in that** the material which serves as crystal nucleus for the at least one pharmaceutically active agent comprises amorphous, pyrogenic silicon dioxide.

4. The oral thin film according to any one of the preceding claims, **characterised in that** the at least one polymer comprises a water-soluble and/or waterswellable polymer.

5. The oral thin film according to any one of the preceding claims, **characterised in that** the at least one polymer is selected from the group consisting of starch, dextrans, carboxymethyl cellulose, hydroxypropyl cellulose, hydroxyethyl cellulose, hydroxypropyl methylcellulose, hydroxypropyl ethyl cellulose, sodium carboxymethyl cellulose, ethyl or propyl cellulose, polyacrylic acids, polyacrylates, polyvinylpyrrolidones, polyvinyl alcohols, polyethylene oxide polymers, polyacrylamides, polyethylene glycols, gelatines, collagen, alginates, pectin, pullulan, tragacanth, chitosan, alginic acid, arabinogalactan, galactomannan, agar, agarose, carrageenan, natural gums, and mixtures thereof.

6. The oral thin film according to any one of the preceding claims, **characterised in that** the at least one polymer is contained in the oral thin film in an amount of 30 to 80 wt.% in relation to the total weight of the oral thin film.

7. The oral thin film according to any one of the preceding claims, **characterised in that** the pharmaceutically active agent is selected from the group consisting of hypnotics, sedatives, antiepiletics, analeptics, psychoneurotropic drugs, neuro-muscle blockers, antspasmodics, antihistamines, antiallergics, cardiotonics, antiarrhythmics, diuretics, hypotensives, vasopressors, antidepressants, antitussives, expectorants, thyroid hormones, sexual hormones, antidiabetics, antitumour active agents, antibiotics, chemotherapeutics and narcotics.

8. The oral thin film according to any one of the preceding claims, **characterised in that** the at least one pharmaceutically active agent comprises ketamine, preferably (S) ketamine or a pharmaceutically acceptable salt thereof.

9. The oral thin film according to any one of the preceding claims, **characterised in that** the oral thin film further comprises at least one auxiliary selected from the group comprising colouring agents, flavourings, sweeteners, tastemasking agents, emulsifiers, enhancers, pH regulators, humectants, preservatives and/or antioxidants.

10. A method for producing an oral thin film according to any one of claims 1 to 9, comprising the steps of:
a) producing a suspension or solution, comprising the at least one polymer, the at least one pharmaceutically active agent and the material which serves as crystal nucleus for the at least one pharmaceutically active agent, and
b) spreading and drying the suspension or solution obtained in step a) in order to obtain a thin film, preferably with a mass per unit area of approximately 50 to 300 g/m².

11. The method according to claim 10, wherein the at least one pharmaceutically active agent in step a) is present substantially completely dissolved.

12. The method according to any one of claims 10 to 11, wherein the at least one pharmaceutically active agent, during the drying in step b), crystallises until below the saturation limit.

13. An oral thin film obtainable by the method according to any one of claims 10 to 12.

14. An oral thin film according to any one of claims 1 to 9 and 13 for use as a medicament.

## Revendications

1. Film mince d'hygiène bucco-dentaire comprenant au moins un polymère et au moins un principe actif pharmaceutiquement actif, dans lequel la concentration de l'au moins un principe actif pharmaceutiquement actif dans le film mince d'hygiène bucco-dentaire est plus importante que la concentration de saturation de l'au moins un principe actif pharmaceutiquement actif dans le film mince d'hygiène bucco-dentaire, **caractérisé en ce que** le film mince d'hygiène bucco-dentaire comprend au moins un matériau, qui fait office de germe de cristallisation pour l'au moins un principe actif pharmaceutiquement actif.

2. Film mince d'hygiène bucco-dentaire selon la revendication 1, **caractérisé en ce que** le matériau, qui fait office de germe de cristallisation pour l'au moins un principe actif pharmaceutiquement actif, est contenu dans le film mince d'hygiène bucco-dentaire en une quantité de 0,01 à 20 % en poids par rapport au poids total du film mince d'hygiène bucco-dentaire.

3. Film mince d'hygiène bucco-dentaire selon l'une quelconque des revendications précédentes, **caractérisé en ce que** le matériau, qui fait office de germe de cristallisation pour l'au moins un principe actif pharmaceutiquement actif, comprend du dioxyde de silicium pyrogène amorphe.

4. Film mince d'hygiène bucco-dentaire selon l'une quelconque des revendications précédentes, **caractérisé en ce que** l'au moins un polymère comprend un polymère soluble dans l'eau et/ou pouvant gonfler dans l'eau.

5. Film mince d'hygiène bucco-dentaire selon l'une quelconque des revendications précédentes, **caractérisé en ce que** l'au moins un polymère est choisi parmi le groupe constitué d'amidon, de dextranes, de cellulose de carboxyméthyle, d'hydroxypropylcellulose, d'hydroxyéthylcellulose, d'hydroxypropylméthylcellulose, d'hydroxypropyléthylcellulose, de cellulose de carboxyméthyle sodique, de cellulose d'éthyle ou de propyle, d'acides polyacryliques, de polyacrylates, de polyvinylpyrrolidones, d'alcools polyvinyliques, de polymères d'oxyde de polyéthylène, de polyacrylamides, de glycols de polyéthylène, de gélatine, de collagène, d'alginates, de pectine, de pullulan, de gomme adragante, de chitosan, d'acide alginique, d'arabinogalactane, de galactomannane, d'agar, d'agarose, de carraghénane, de gommes naturelles ainsi que de mélanges de ceux-ci.

6. Film mince d'hygiène bucco-dentaire selon l'une quelconque des revendications précédentes, **caractérisé en ce que** l'au moins un polymère est contenu dans le film mince d'hygiène bucco-dentaire en une quantité de 30 à 80 % en poids par rapport au poids total du film mince d'hygiène bucco-dentaire.

7. Film mince d'hygiène bucco-dentaire selon l'une quelconque des revendications précédentes, **caractérisé en ce que** le principe actif pharmaceutiquement actif est choisi parmi le groupe constitué d'hypnotiques, de sédatifs, d'anti-épileptiques, de weckamines, d'antipsychotiques, de bloquants neuromusculaires, d'antispasmodiques, d'antihistaminiques, d'antiallergiques, de cardiotoniques, d'anti-arythmiques, de diurétiques, d'hypotenseurs, de vasopresseurs, d'antidépresseurs, d'antitussifs, d'expectorants, d'hormones thyroïdiennes, d'hormones sexuelles, d'antidiabétiques, de principes actifs antitumoraux, d'antibiotiques, d'agents de chimiothérapie et de narcotiques.

8. Film mince d'hygiène bucco-dentaire selon l'une quelconque des revendications précédentes, **caractérisé en ce que** l'au moins un principe actif pharmaceutiquement actif comprend de la kétamine, de préférence de la S-kétamine, ou un sel pharmaceutiquement acceptable de celle-ci.

9. Film mince d'hygiène bucco-dentaire selon l'une quelconque des revendications précédentes, **caractérisé en ce que** le film mince d'hygiène bucco-dentaire comprend en outre au moins un excipient choisi parmi le groupe comprenant des colorants, des substances aromatiques, des édulcorants, des agents masquant le goût, des agents émulsifiants, des agents activateurs, des régulateurs de pH, des agents humectants, des agents de conservation et/ou des agents antioxydants.

10. Procédé pour fabriquer un film mince d'hygiène bucco-dentaire selon l'une quelconque des revendications 1 à 9, comprenant les étapes :
a) de fabrication d'une suspension ou d'une solution, comprenant l'au moins un polymère, l'au moins un principe actif pharmaceutiquement actif, et le matériau qui fait office de germe de cristallisation pour l'au moins un principe actif pharmaceutiquement actif, et
b) d'étalement et de séchage de la suspension ou de la solution obtenue à l'étape a) pour obtenir un film mince, de préférence avec un poids surfacique d'environ 50 à 300 g/m².

11. Procédé selon la revendication 10, dans lequel l'au moins un principe actif pharmaceutiquement actif est présent dans l'étape a) sous une forme sensiblement totalement dissoute.

12. Procédé selon l'une quelconque des revendications 10 à 11, dans lequel l'au moins un principe actif pharmaceutiquement actif se cristallise lors du séchage dans l'étape b) jusque sous la limite de saturation.

13. Film mince d'hygiène bucco-dentaire pouvant être obtenu selon le procédé selon l'une quelconque des revendications 10 à 12.

14. Film mince d'hygiène bucco-dentaire selon l'une quelconque des revendications 1 à 9 et 13 destiné à être utilisé en tant que médicament.
